# EUROPEAN PATENT SPECIFICATION

(11) **EP 0 772 429 B1**
(45) Date of publication and mention of the grant of the patent: **02.12.1998**
(21) Application number: 95923047.5
(22) Date of filing: 13.06.1995
(51) Int. Cl.: A61F 13/62

(54) **REFASTENABLE STRETCHABLE FASTENER SYSTEM**
WIEDERBEFESTIGBARES, ELASTISCHES BEFESTIGUNGSSYSTEM
SYSTEME D'ATTACHE ETIRABLE ET REUTILISABLE

(30) Priority: 26.07.1994 US 281199
(43) Date of publication of application: 14.05.1997
(73) Proprietor: FIBERWEB NORTH AMERICA, INC., Simpsonville, SC 29681 (US)
(72) Inventor: AUSTIN, Jared Asher, Greer, SC 29650 (US)
(74) Representative: Orès, Bernard
(86) International application number: US9507495
(87) International publication number: WO9603101

(56) References cited:
- EP-A- 0 418 493
- EP-A- 0 605 013
- GB-A- 2 169 930
- US-A- 2 133 840
- US-A- 4 705 710
- US-A- 5 125 246

## Description

### Field of the Invention

This invention relates to a refastenable stretchable fastener which can be used to secure two objects together such that their relative positions can change while they remain secured to one another. This fastener has particular application as a garment closure system which is capable of providing substantial freedom of movement for the wearer of the garment.

### Background of the Invention

The general concept of a hook and loop fastening system is known from the prior art. A variety of hook and loop fastener systems are available commercially from Velcro, Inc., Manchester, New Hampshire and from 3M Corporation, St. Paul, Minnesota. The loop portion is generally configured with a relatively soft, felt-like texture whereas the hook portion is specifically designed with a certain degree of stiffness to enable the hooks to penetrate the loops and to releasably engage them. Conventional hook and loop fasteners form a relatively inextensible connection when fastened together. The desirability of having some degree of extensibility in the connection formed by a hook and loop fastener system for use in certain applications has long been recognized. Thus for example, an elastic belt with hook and loop fasteners at its ends was the subject of US Patent 3,086,529. This device is designed to control the flow of blood when it is drawn from an appendage. The use of an elastic belt with hook and loop fasteners at its ends is used to secure a disposable diaper in US Patent 4,617,022. A hook and loop fastener is incorporated in a disposable diaper with elastic ears in European Patent Application EP 433,951. In this diaper, a hook fastener is placed at the end of the elastic ear. The loop member is placed on the outer surface of the opposite side of the diaper, so that when the diaper is placed on the infant, there is some flexibility in the circumference of the diaper, allowing it to conform to body movements. In all the above cases, the extensibility is provided by a belt or by an elastic fabric; it is not provided by either the hook member or the loop member of the attachment system.

Other attempts to impart extensibility to the connection formed by a hook and loop fastener system include the use of elastomeric yarns in various knit constructions, such as that discussed in U.S. Patent Nos. 4,705,710 and 5,125,246.

### Summary of the Invention

The present invention provides a refastenable mechanical fastener system which gives a secure, but extensible connection between two components of the fastener system. The fastener system is particularly suited for providing comfort and conformability for the wearer of a garment or other article utilizing the fastener system.

One member of the fastener system is an engaging component with a plurality of engaging elements in the form of projections. The projections, or "engaging elements", may have any shape such as hooks, T's, mushrooms, or any other shape which is well known in the art. An example of such a member is the hook component of a Velcro fastener system. The other member of the fastener system is a cooperating receiving component which is extensible in at least one direction and has on its surface apertures or loops capable of mechanically engaging the projections of the engaging component. This second member is an elastomeric net. The net comprises two or more sets of parallel strands which may be perpendicular to one another. A preferred configuration is two sets of parallel strands which are perpendicular to one another. The interstices between the strands engage the projections of the first member. A second specific embodiment comprises an elastic net which has been interpenetrated by textile fibers so that the fibers are located in the interstices between the strands and cover the upper and lower surfaces of the net. The fibers are secured to the net and to each other by mechanical interlocking, which can be provided by a suitable process such as by hydroentanglement or needlepunching. The mechanical interlocking of the fibers forms loops of various sizes. These loops are capable of engaging the projections of the first fastener member, which in this case might be in the form of hooks.

A particularly useful application of this invention is in absorbent articles such as disposable diapers, incontinence briefs, diaper holders, and the like. The present invention provides elasticity in the waist of the assembled diaper for comfort and freedom of movement of the wearer. An advantage of this embodiment over prior art (e.g. US Patent 4,617,022 and European patent Application EP 433,951) is that previous flexible closure systems have required at least three elements: the two members of the attachment system and at least one piece of elastic fabric attached to at least one of the members. In the present invention, the elastic fabric becomes one of the two members of the attachment system, reducing the complexity of providing such attachment systems for disposable absorbent articles.

### Brief Description of the Drawings

Some of the features of the invention having been stated, others will become apparent from the detailed description which follows, and from the accompanying drawings, in which --
Figure 1 is a perspective view of a disposable diaper utilizing the attachment system of this invention;
Figure 2 is a fragmentary detailed view of a diaper with an attachment system in accordance with one embodiment of the invention, shown in the unfastened state;
Figure 3 is a fragmentary detailed view showing the attachment system of Figure 2 in the fastened state; and
Figure 4 is a cross sectional view of the attachment system taken substantially along the line 4-4 of Figure 3.

### Detailed Description

The mechanical fastener system of this invention includes two members: an engaging component of a relatively inextensible material with small projections or engaging elements protruding from a surface, and a cooperating receiving component which is elastically extensible and formed from an elastomeric net. The net may be surrounded and interpenetrated by inelastic fibers.

The net can be manufactured by a variety of methods. The formation of a net by embossing an extruded film is one such method. The process disclosed in US patent 3,954,933 is suitable for manufacturing this type of net. The process disclosed in US Patent 4,329,309 is also suitable for manufacturing this net. The die extrusion processes taught in US Patents 3,252,181 and 3,384,692 are especially suitable methods. In the die extrusion method, a plurality of parallel longitudinal strands are extruded and a plurality of parallel transverse strands are extruded perpendicular to the longitudinal strands at spaced intervals. One or both of the longitudinal or transverse groups of strands are formed of an elastomeric material.

The elastic strands may be formed of a thermoplastic or crosslinked elastomeric material. Suitable thermoplastic elastomers include the diblock, triblock, radial and star copolymers based on polystyrene (S) and unsaturated or fully hydrogenated rubber blocks. The rubber block can consist of butadiene (B), isoprene (I), or the hydrogenated version, ethylene-butylene (EB). For example, S-B, S-I, S-EB, as well as S-B-S, S-I-S, S-EB-S linear block copolymers can be used. Typically when used one or more of the diblock copolymers are blended with the triblock or radial copolymer elastomers. Preferred thermoplastic elastomers of this type can include the KRATON polymers sold by Shell Chemical Company or the VECTOR polymers sold by DEXCO. Other elastomeric thermoplastic polymers include polyurethane elastomeric materials such as ESTANE sold by BF Goodrich Company; polyester elastomers such as HYTREL sold by E. I. Du Pont De Nemours Company; polyetherester elastomeric materials such as ARNITEL sold by Akzo Plastics; and polyetheramide elastomeric materials such as PEBAX sold by ATO Chemie Company; and the like.

The elastic strands in the elastomeric net can also be prepared from blends of thermoplastic elastomers with other polymers such as polyolefin polymers, e.g. blends of KRATON polymers with polyolefins such as polypropylene and polyethylene, and the like. These polymers can provide lubrication and decrease melt viscosity, allow for lower melt pressures and temperatures and/or increase throughput, and provide better bonding properties. In a particularly preferred embodiment of the invention, polymers can be included in the blend as a minor component, for example in an amount of from about 5% by weight up to about 50% by weight, preferably from about 10 to about 30% by weight. Suitable thermoplastic materials include poly(ethylene-vinyl acetate) polymers having an ethylene content of up to about 50% by weight, preferably between about 15 and about 30% by weight, and copolymers of ethylene and acrylic acid or esters thereof, such as poly(ethylene-methyl acrylate) or poly(ethylene-ethyl acrylate) wherein the acrylic acid or ester component ranges from about 5 to about 50% by weight, preferably from about 15 to 30% by weight.

In one embodiment of this invention, the strands of the elastic net provide the apertures, or loops, into which the engaging elements of the engaging component are inserted. In a second embodiment, a composite of an elastic net and textile staple fibers is employed as the receiving component into which the engaging elements are inserted. In this embodiment, the loops are provided by the staple fibers or by a combination of the staple fibers and the strands of the net. In this second embodiment, the composite is produced by causing the staple fibers to interpenetrate and surround the strands of the net. This integration of the fibers and the net can be accomplished by hydroentangling, as disclosed in US Patent 4,775,579 or by needlepunching.

A variety of textile fibers are useful in this invention. Fibers which are easily carded into webs which can be hydroentangled or needlepunched are preferred. Staple fibers from polyester and polypropylene polymers are inexpensive and work well in this application. However, nylon, rayon, cotton, or wool also can be employed. Blends of staple fibers may also be employed. Fiber deniers* from 1.0 to 15 can be employed, but to best practice the invention the properties of the textile fibers are tailored to provide the most secure attachment with the engaging elements. The higher denier fibers are best used with engaging elements which are relatively coarse. Staple fiber lengths ranging from one to six inches* are most suitable. Below one inch length the propensity for the fibers to form loops is diminished. Above 6 inches length, the fiber becomes difficult to force into the apertures of the net.
* 1 den = 1,1dtex
* 1 inch = 25.4mm

The fiber component may have a basis weight of from about 50 to about 500 grams per square meter. The net component may have a basis weight ranging from about 20 grams per square meter to about 300 grams per square meter, more preferably from about 35 to about 200 grams per square meter and can employ strands having diameters greater than 50 microns, and preferably greater than 200 microns. The mesh size of the elastomeric net can vary from about 4x4 to about 20x20 strands per square inch**.
** 1 square inch = 6.45cm²

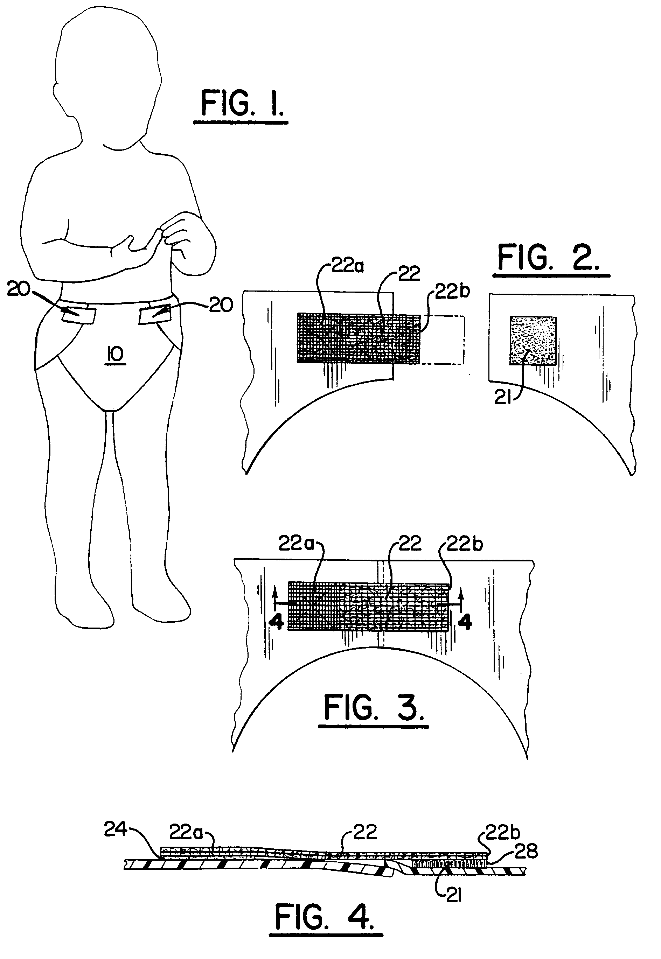

Figure 1 depicts a typical disposable diaper **10** of the type with which the present invention can be utilized. The disposable diaper includes a diaper body which is typically comprised of a number of layers, including a moisture permeable inner layer for contacting the skin of the wearer, a moisture impermeable outer layer or liner, and an interior absorbent member for absorbing fluids. For purposes of the present invention, the detailed construction of the diaper body is not critical, and can be in accordance with any of a number of known commercially available constructions, as exemplified for example in U.S. Patent 4,704,115.

The diaper **10** shown in Figure 1 is in the general shape of an hourglass and includes relatively wide front and rear portions adapted to be positioned along the front and rear of the wearer and to form front and rear waistbands, and a relatively narrower middle portion which is adapted to be positioned in the crotch area of the wearer. A mechanical fastener system, generally indicated at **20** in Figure 1, is provided on each side of the diaper for securing the front and rear portions of the diaper together so that the diaper conforms to the waist of the wearer.

As seen more clearly in Figure 2, the mechanical fastener system **20** includes a first fastener component **21** secured to one portion of the diaper body and a tab **22** secured to an opposing second portion of the diaper body by suitable means such as an adhesive or heat sealing. The first fastener component **21** is an engaging component and includes a base or support and a plurality of upstanding engaging elements, shown at **28** in Figure 4, extending from the base. The engaging elements **28** may be in the form of hooks, T's, mushrooms, or other shapes as are well known in the art.

The tab **22** has a first end portion **22a** secured to the diaper body by suitable means, such as an adhesive layer **24**, and a free end portion **22b** extending from the diaper body and forming a cooperating receiving component of the mechanical fastener system adapted to fasten to the engaging component **21**.

The tab **22** is of a stretchable elastic construction which allows the diaper to be fastened together while providing elasticity in the waist of the assembled diaper to provide comfort and freedom of movement for the wearer. More particularly, the tab **22** is elastic in the length or longitudinal direction. The crosswise or width dimension of the tab may or may not have elasticity. In a preferred embodiment, the tab has elasticity only in the longitudinal direction but not in the crosswise direction so that the tab can be stretched in the length direction without necking down in the width direction.

The tab **22**, more particularly, comprises an elastic net, including intersecting and interconnected longitudinal strands and transverse or crosswise strands. The longitudinal strands are formed from an elastic material, while the transverse strands are relatively inelastic. In the embodiment shown, the tab also includes a fibrous layer of textile fibers on at least one side of the net.

### EXAMPLE

The following example more fully illustrates the extensible tab **22** or receiving component and how it can be made.

Two extensible fastener members were prepared. Sample A employed an extruded net having 6 strands per inch of styrenic elastomer in the machine direction and 9 strands per inch of styrenic elastomer in the cross machine direction. Sample A had a basis weight of 170 grams per square meter. Sample B employed an extruded net having 18 strands per inch of styrenic elastomer in the machine direction and 10 strands per inch of styrenic elastomer in the cross machine direction. Sample B had a basis weight of 181 grams per square meter. Both extruded nets were obtained from the Conwed Corporation.

A carded web of 24 grams per square meter basis weight was prepared from a blend of 70% Hercules T-182 polypropylene staple fiber and 30% Hoechst K-54 polyester/polyethylene bicomponent staple fiber. To prepare the extensible fastener members, the extruded nets were placed on top of two layers of carded web. Two additional card webs were placed on top of the nets. These layered structures were passed through a Dilo needlepunching machine outfitted with 15/18 40/3 C-222-62027 Groz-Beckert needles. The structures were needlepunched on both sides at a density of 90 penetrations per square centimeter. The cross direction mechanical properties of these samples are given in Table I.

These extensible fastener members were tested with two types of hook attachment members: a Velcro hook attachment and a 3M Scotchmate hook attachment. These attachment members were attached to polyethylene sheet by epoxy adhesive. A piece of extensible fastener member 1" wide by 3" long was cut such that the cross machine direction ran parallel to the side which was 3" long. The hook attachment was pressed into one end of the extensible fastener member using the normal force of the thumb. The joined members were placed between the jaws of an Instron tester. The Instron tester was set in motion at a crosshead speed of 127 mm/min and the peak force and the elongation at peak force recorded. These are given in Table II, normalized to the area of the hook member. Also given in Table II are the peak load and elongation at peak force for the tape attachment systems of two brands of disposable diaper. The attachment systems taught by this invention clearly give a greater amount of extension at a much lower force than the inelastic tape systems.

**TABLE I**

| CROSS DIRECTION MECHANICAL PROPERTIES EXTENSIBLE FASTENING MEMBER | | | |
|---|---|---|---|
| | Maximum Force g per cm* | Elongation at Maximum Force % | Elongation to Break % |
| Sample A | 818 | 181 | 284 |
| Sample B | 905 | 195 | 293 |

| | | | |
|---|---|---|---|
| * 1 g/cm = 0,0098 N/cm | | | |

**TABLE II**

| ATTACHMENT FORCE TEST | | |
|---|---|---|
| | Maximum Force g per sq. cm.* of attachment area | Elongation at Maximum Force % |
| Sample A/Velcro Hook | 173 | 171 |
| Sample B/Velcro Hook | 239 | 165 |
| Sample A/Scotchmate Hook | 260 | 236 |
| Sample B/Scotchmate Hook | 214 | 176 |
| Huggies Ultra Trim Tape Attachment | 622 | 42 |
| Drypers Tape Attachment | 683 | 6 |

| | | |
|---|---|---|
| * 1 g/sq.cm = 0,0098 N/sq.cm | | |

## Claims

1. A refastenable mechanical fastener system (20) comprising an engaging component (21) including a plurality of engaging elements (28) and a cooperating receiving component (22) having a surface which releasably and refastenably couples with the engaging elements (28) of said engaging component (21), whereby said cooperating receiving component (22) comprising an elastomeric net comprising a plurality of thermally fused intersecting polymeric strands or a perforated polymeric film, and said receiving component (22) being elastically extensible in at least one direction.

2. The fastening system (20) of claim 1, wherein said elastomeric net comprises intersecting longitudinal and transverse strands.

3. The fastening system (20) of claim 2, wherein the longitudinal strands of said net are formed of an elastomeric material, and said transverse strands are formed of a non-elastomeric material, and said receiving component (22) is stretchable in the length direction without necking down in the width direction upon stretching.

4. The fastening system (20) of claim 1 wherein said receiving component (22) additionally includes a layer of fibers positioned on at least one side of said net.

5. A garment employing the fastening system (20) described in claim 1.

6. A refastenable mechanical fastener system (20) comprising a relatively inextensible engaging component (21) including a plurality of engaging elements (28), and a cooperating elastically extensible receiving component (22) which releasably and refastenably couples with the engaging elements (28) of said engaging component (21), whereby said receiving component (22) comprising a nonwoven fabric including an elastomeric net comprising a plurality of thermally fused intersecting polymeric strands or a perforated polymeric film and textile staple fibers interpenetrating said elastomeric net.

7. A disposable diaper (10) comprising a diaper body and a refastenable mechanical fastener system (20) carried by the diaper body for fastening together portions of the diaper body to secure the diaper around the waist of a wearer, the mechanical fastener system (20) including an engaging component (21) carried by one portion of the diaper body, said engaging component (21) including a plurality of engaging elements (28), and a cooperating receiving component (22) carried by another portion of the diaper body having a surface which releasably and refastenably couples with the engaging elements (28) of said engaging component (1), whereby said receiving component (22) comprising an elastomeric net comprising a plurality of thermally fused intersecting polymeric strands or a perforated polymeric film, and said receiving component (22) being elastically extensible in at least one direction.

8. A disposable diaper (10) according to claim 7, wherein said receiving component (22) comprises a stretchable elastic tab having opposite end portions (22a, 22b), one end portion (22a) of said elastic tab being secured to said portion of the diaper body, and the tab having a free end portion (22b) extending from the diaper body and adapted to cooperatively engage with said engaging fastener component (21) to secure the portions of the diaper body to one another in assembled relation, said stretchable elastic tab providing elasticity in the waist of the assembled diaper for comfort and freedom of movement of the wearer.

9. A disposable diaper (10) according to claim 7, wherein said elastomeric net is formed of intersecting longitudinal and transverse strands.

10. A disposable diaper (10) according to claim 9, wherein said longitudinal strands are formed of an elastomeric material, and said crosswise strands are formed of a non-elastomeric material, and said tab is stretchable in the length direction without necking down in the width direction upon stretching.

## Patentansprüche

1. Wiederbefestigbares mechanisches Befestigungssystem (20), welches eine Eingriffskomponente (21), mit einer Vielzahl von Eingriffselementen (28) und eine damit zusammenwirkende Aufnahmekomponente (22) mit einer Oberfläche aufweist, die sich lösbar und wieder befestigbar mit den Eingriffselementen (28) der Eingriffskomponente (21) verbindet, wobei die damit zusammenwirkende Aufnahmekomponente (22) ein elastomeres Netz aufweist, welches eine Vielzahl von thermisch verschmolzenen sich kreuzenden polymeren Strängen oder einen perforierten polymeren Film aufweist, und wobei die Aufnahmekomponente (22) in zumindest eine Richtung elastisch dehnbar ist.

2. Befestigungssystem (20) nach Anspruch 1, wobei das elastomere Netz sich kreuzende Längs- und Querstränge aufweist.

3. Befestigungssystem (20) nach Anspruch 2, wobei die Längsstränge des Netzes aus einem elastomeren Material gebildet sind, und wobei die Querstränge aus einem nicht elastomeren Material gebildet sind, und wobei die Aufnahmekomponente (22) in Längsrichtung dehnbar ist, ohne daß eine Einschnürung in Richtung der Breite während der Dehnung auftritt.

4. Befestigungssystem (20) nach Anspruch 1, wobei die Aufnahmekomponente (22) zusätzlich eine Schicht von Fasern aufweist, welche an zumindest einer Seite des Netzes angeordnet sind.

5. Kleidungsstück, bei welchem ein Befestigungssystem (20) nach Anspruch 1 verwendet wird.

6. Wiederbefestigbares mechanisches Befestigungssystem (20), welches eine relativ undehnbare Eingriffskomponente (21) mit einer Vielzahl von Eingriffselementen (28) und eine damit zusammenwirkende plastische dehnbare Aufnahmekomponente (22) aufweist, welche sich lösbar und wiederbefestigbar mit den Eingriffselementen (28) der Eingriffskomponente (21) verbindet, wobei die Aufnahmekomponente (22) einen nicht gewebten Stoff mit einem elastomeren Netz aufweist, welches eine Vielzahl von thermisch verschmolzenen sich kreuzenden polymeren Strängen oder einen perforierten polymeren Film und Gewebestapelfasern aufweist, welche das elastomere Netz durchdringen.

7. Wegwerfwindel (10), welche einen Windelkörper und ein wiederbefestigbares mechanisches Befestigungssystem (20) aufweist, das an dem Windelkörper angebracht ist, um Teile des Windelkörpers miteinander zu befestigen, so daß die Windel um die Taille eines Trägers gesichert ist, wobei das mechanische Befestigungssystem (20) eine Eingriffskomponente (21), welche an einem Teil des Windelkörpers angebracht ist, wobei die Eingriffskomponente (21) eine Vielzahl von Eingriffselementen (28) aufweist, und eine damit zusammenwirkende Aufnahmekomponente (22), welche an einem anderen Abschnitt des Windelkörpers angebracht ist, mit einer Oberfläche aufweist, die sich lösbar und wiederbefestigbar mit den Eingriffselementen (28) der Eingriffskomponente (21) verbindet, wobei die Aufnahmekomponente (22) ein elastomeres Netz mit einer Vielzahl von thermisch verschmolzenen sich kreuzenden polymeren Strängen oder einen perforierten polymeren Film aufweist, und wobei die Aufnahmekomponente (22) in zumindest einer Richtung elastisch dehnbar ist.

8. Wegwerfwindel (10) nach Anspruch 7, wobei die Aufnahmekomponente (22) einen dehnbaren elastischen Vorsprung mit sich gegenüberliegenden Endabschnitten (22a, 22b) aufweist, wobei ein Endabschnitt (22a) des elastischen Vorsprungs an dem Abschnitt des Windelkörpers befestigt ist und der Vorsprung einen freien Endabschnitt (22b) aufweist, welcher sich von dem Windelkörper erstreckt und fähig ist, gemeinsam mit der Eingriffsbefestigungskomponente (21) im Eingriff zu sein, um die Abschnitte des Windelkörpers im zusammengefügten Zustand miteinander zu befestigen, wobei der dehnbare elastische Vorsprung für Elastizität in der Taille der zusammengefügten Windel und für Komfort und Bewegungsfreiheit des Trägers sorgt.

9. Wegwerfwindel (10) nach Anspruch 7, wobei das elastomere Netz aus sich kreuzenden Längssträngen und Quersträngen gebildet ist.

10. Wegwerfwindel (10) nach Anspruch 9, wobei die Längsstränge aus einem elastomeren Material gebildet sind und die quer verlaufenden Stränge aus einem nicht elastomeren Material gebildet sind, und wobei der Vorsprung in Längsrichtung dehnbar ist, ohne daß eine Einschnürung in Richtung der Breite während der Dehnung auftritt.

## Revendications

1. Système d'attache mécanique réattachable (20) comprenant un composant d'accouplement (21), qui comporte une pluralité d'éléments d'accouplement (28), et un composant de réception coopérant (22) qui présente une surface venant en prise de façon détachable et réattachable avec les éléments d'accouplement (28) dudit composant d'accouplement (21), dans lequel ledit composant de réception coopérant (22) comprend une toile maillée en élastomère comportant une pluralité de fils de polymère thermiquement soudés en intersection ou un film de polymère perforé, et ledit composant de réception (22) est élastiquement extensible dans au moins une direction.

2. Système d'attache (20) selon la revendication 1, dans lequel ladite toile maillée en élastomère comprend des fils longitudinaux et transversaux en intersection.

3. Système d'attache (20) selon la revendication 2, dans lequel les fils longitudinaux de ladite toile maillée sont en une matière élastomère et lesdits fils transversaux sont en une matière non élastomère, et ledit composant de réception (22) est étirable dans la direction de la longueur sans rétrécissement dans la direction de la largeur lors de l'étirage.

4. Système d'attache (20) selon la revendication 1, dans lequel ledit composant de réception (22) comprend additionnellement une couche de fibres placées sur au moins une face de ladite toile maillée.

5. Vêtement employant le système d'attache (20) selon la revendication 1.

6. Système d'attache mécanique réattachable (20) comprenant un composant d'accouplement relativement inextensible (21) qui comporte une pluralité d'éléments d'accouplement (28), et un composant de réception coopérant élastiquement extensible (22) qui vient en prise de façon détachable et réattachable avec les éléments d'accouplement (28) dudit composant d'accouplement (21), dans lequel ledit composant de réception (22) comprend une étoffe non-tissée qui inclut une toile maillée en élastomère, constituée d'une pluralité de fils de polymère thermiquement soudés en intersection ou d'un film de polymère perforé, et des fibres textiles discontinues interpénétrant ladite toile maillée en élastomère.

7. Couche jetable (10) comprenant un corps de couche et un système d'attache mécanique réattachable (20) porté par le corps de couche pour attacher ensemble des parties du corps de couche afin de fixer la couche autour de la taille d'un porteur, le système d'attache mécanique (20) comprenant un composant d'accouplement (21) porté par une partie du corps de couche, ledit composant d'accouplement (21) incluant une pluralité d'éléments d'accouplement (28), et un composant de réception coopérant (22) porté par une autre partie du corps de couche et présentant une surface qui vient en prise de façon détachable et réattachable avec les éléments d'accouplement (28) dudit composant d'accouplement (1), dans laquelle ledit composant de réception (22) comprend une toile maillée en élastomère qui est constituée d'une pluralité de fils de polymère thermiquement soudés en intersection ou d'un film de polymère perforé, et ledit composant de réception (22) est élastiquement extensible dans au moins une direction.

8. Couche jetable (10) selon la revendication 7, dans laquelle ledit composant de réception (22) comprend une patte élastique étirable ayant des parties d'extrémité opposées (22a,22b), une partie d'extrémité (22a) de ladite patte élastique étant fixée à ladite partie du corps de couche, et la patte ayant une partie d'extrémité libre (22b) qui s'étend à partir du corps de couche et peut venir en prise de façon coopérante avec ledit composant d'accouplement de l'attache (21) afin de fixer les parties du corps de couche l'une à l'autre en relation assemblée, ladite patte élastique étirable procurant une élasticité de la taille de la couche assemblée pour le confort et la liberté de mouvement du porteur.

9. Couche jetable (10) selon la revendication 7, dans laquelle ladite toile maillée en élastomère est formée de fils longitudinaux et transversaux en intersection .

10. Couche jetable (10) selon la revendication 9, dans laquelle lesdits fils longitudinaux sont en une matière élastomère et lesdits fils transversaux sont en une matière non élastomère, et ladite patte est étirable dans la direction de la longueur sans rétrécissement dans la direction de la largeur lors de l'étirage.
